(19)

**Europäisches Patentamt**

**European Patent Office**

**Office européen des brevets**

(11)  **EP 4 706 547 A1**

(12) **EUROPEAN PATENT APPLICATION**

(43) Date of publication:
**11.03.2026 Bulletin 2026/11**

(21) Application number: **25197266.7**

(22) Date of filing: **21.08.2025**

(51) International Patent Classification (IPC):
**A61B 8/08** *(2006.01)*    **A61B 8/00** *(2006.01)*
**G06T 7/00** *(2017.01)*    **G16H 50/30** *(2018.01)*

(52) Cooperative Patent Classification (CPC):
**A61B 8/0883; A61B 8/065; A61B 8/463;
A61B 8/465; A61B 8/5223; G06T 7/0016;
G16H 50/30;** A61B 8/469

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **09.09.2024 JP 2024155085**

(71) Applicant: **FUJIFILM Corporation
Tokyo 106-8620 (JP)**

(72) Inventor: **RAGHAVA, Krishnan
Kaisei (JP)**

(74) Representative: **Meissner Bolte Partnerschaft
mbB
Patentanwälte Rechtsanwälte
Postfach 86 06 24
81633 München (DE)**

(54) **ULTRASOUND DIAGNOSTIC APPARATUS AND METHOD OF CONTROLLING ULTRASOUND DIAGNOSTIC APPARATUS**

(57)    Provided are an ultrasound diagnostic apparatus capable of easily calculating an ejection fraction of a cavity of a heart in a short time and a method of controlling the ultrasound diagnostic apparatus.

An ultrasound diagnostic apparatus includes: an image acquisition unit that acquires first ultrasound images in which a first cross section of a heart is imaged, and second ultrasound images in which a second cross section of the heart is imaged; a cross-sectional area calculation unit that calculates a cross-sectional area of a cavity of the heart for the first ultrasound images and the second ultrasound images; a cross-sectional area change curve generation unit that generates a first cross-sectional area change curve of the first cross section and a second cross-sectional area change curve of the second cross section; an alignment unit that aligns the first cross-sectional area change curve and the second cross-sectional area change curve with each other; and a frame update unit that, in a case in which one frame of the first ultrasound image or the second ultrasound image is updated based on an instruction from a user, automatically updates the other frame.

EP 4 706 547 A1

**Description**

**BACKGROUND OF THE INVENTION**

1. Field of the Invention

The present invention relates to an ultrasound diagnostic apparatus that captures an ultrasound image of a heart of a subject and a method of controlling the ultrasound diagnostic apparatus.

2. Description of the Related Art

In the related art, it is known that an ultrasound image representing a tomographic plane of a heart of a subject is captured using a so-called ultrasound diagnostic apparatus, and an ejection fraction of a cavity of the heart, such as a so-called left ventricular ejection fraction (LVEF), is calculated using the ultrasound image. In a case of calculating the ejection fraction of the cavity of the heart, an ultrasound image representing end-systolic and end-diastolic states of the heart in a so-called apical two-chamber cross section and an ultrasound image representing end-systolic and end-diastolic states of the heart in a so-called apical four-chamber cross section are often used.

In order to easily obtain an ultrasound image suitable for calculating the ejection fraction of the cavity of the heart, for example, an ultrasound diagnostic apparatus as disclosed in JP2004-073850A has been developed. The ultrasound diagnostic apparatus disclosed in JP2004-073850A divides a period corresponding to one heartbeat into a systole and a diastole of the heart, aligns time phases of ultrasound images of a plurality of frames representing an apical two-chamber cross section and ultrasound images of a plurality of frames representing an apical four-chamber cross section in each of the systole and the diastole of the heart, and simultaneously displays two types of ultrasound images whose time phases are aligned with each other.

**SUMMARY OF THE INVENTION**

In order to obtain an ultrasound image suitable for the ejection fraction of the cavity of the heart, a user may compare ultrasound images of a plurality of frames with each other and select an appropriate ultrasound image from among the ultrasound images of the plurality of frames. In this case, the user usually searches for an ultrasound image suitable for calculating the ejection fraction while updating each of the ultrasound image representing the apical two-chamber cross section and the ultrasound image representing the apical four-chamber cross section displayed on a monitor, but, in JP2004-073850A, the update of the ultrasound image by the user is not taken into account, and there is room for improvement in order to shorten a time required for calculating the ejection fraction.

The present invention has been made in order to solve such a problem in the related art, and an object of the present invention is to provide an ultrasound diagnostic apparatus capable of easily calculating an ejection fraction of a cavity of a heart in a short time and a method of controlling the ultrasound diagnostic apparatus.

The above-described object can be achieved with the following configurations.

[1] An ultrasound diagnostic apparatus comprising: an image acquisition unit that acquires first ultrasound images of a plurality of frames which are consecutive in time series and in which a first cross section of a heart of a subject is imaged, and second ultrasound images of a plurality of frames which are consecutive in time series and in which a second cross section of the heart different from the first cross section is imaged; a cross-sectional area calculation unit that calculates a cross-sectional area of a cavity of the heart for each frame for the first ultrasound images of the plurality of frames and the second ultrasound images of the plurality of frames; a cross-sectional area change curve generation unit that generates a first cross-sectional area change curve of the first cross section and a second cross-sectional area change curve of the second cross section, each of which represents a temporal change in the cross-sectional area, based on the cross-sectional area calculated by the cross-sectional area calculation unit; an alignment unit that aligns the first cross-sectional area change curve and the second cross-sectional area change curve, which are generated by the cross-sectional area change curve generation unit, with each other; a monitor that displays the first ultrasound image and the second ultrasound image at corresponding points on the first cross-sectional area change curve and the second cross-sectional area change curve; and a frame update unit that, in a case in which one frame of the first ultrasound image or the second ultrasound image displayed on the monitor is updated based on an instruction from a user, automatically updates the other frame of the first ultrasound image or the second ultrasound image so as to correspond with the one frame.

[2] The ultrasound diagnostic apparatus according to [1], in which the alignment unit includes a curve selection unit that selects a first curved portion and a second curved portion, which include the same number of change cycles, from the first cross-sectional area change curve and the second cross-sectional area change curve, a normalization unit that normalizes the first curved portion and the second curved portion, and a portion alignment unit that aligns the first curved portion and the second curved portion, which are normalized by the normalization unit, with each other.

[3] The ultrasound diagnostic apparatus according to [2], in which the curve selection unit selects the first curved portion and the second curved portion based on sections selected by the user in the first cross-sectional area change curve and the second cross-sectional area change curve.

[4] The ultrasound diagnostic apparatus according to [2], in which the curve selection unit selects the first curved portion or the second curved portion based on a section selected by the user in the first cross-sectional area change curve or the second cross-sectional area change curve, calculates the number of change cycles included in one of the first curved portion or the second curved portion, and selects the other of the first curved portion or the second curved portion based on the calculated number of change cycles.

[5] The ultrasound diagnostic apparatus according to [2], in which the curve selection unit selects the first curved portion and the second curved portion which include the number of change cycles that is the same number as a designated number of cycles designated by the user.

[6] The ultrasound diagnostic apparatus according to [2], in which the normalization unit normalizes the first curved portion and the second curved portion using respective maximum values thereof.

[7] The ultrasound diagnostic apparatus according to [1], in which the alignment unit includes a normalization unit that normalizes the first cross-sectional area change curve and the second cross-sectional area change curve, a similar portion specifying unit that specifies a first curved portion and a second curved portion which are portions most similar to each other in the first cross-sectional area change curve and the second cross-sectional area change curve which are normalized by the normalization unit, and a portion alignment unit that aligns the specified first curved portion and the specified second curved portion with each other.

[8] The ultrasound diagnostic apparatus according to [7], in which the normalization unit normalizes the first cross-sectional area change curve and the second cross-sectional area change curve using respective maximum values thereof.

[9] The ultrasound diagnostic apparatus according to [7], in which the normalization unit normalizes the first cross-sectional area change curve and the second cross-sectional area change curve using respective average values and standard deviations thereof.

[10] The ultrasound diagnostic apparatus according to any one of [1] to [9], further comprising: a frame selection unit that selects, as a first measurement frame group, one set of the first ultrasound images corresponding to a maximal value and a minimal value in the first cross-sectional area change curve, and selects, as a second measurement frame group, one set of the second ultrasound images corresponding to a maximal value and a minimal value in the second cross-sectional area change curve aligned with the maximal value and the minimal value in the first cross-sectional area change curve by the alignment unit.

[11] The ultrasound diagnostic apparatus according to [10], in which the monitor displays the first ultrasound image of the first measurement frame group and the second ultrasound image of the second measurement frame group which are selected by the frame selection unit, and in a case in which one frame of the first ultrasound image or the second ultrasound image displayed on the monitor is updated, the frame update unit updates the other frame of the first ultrasound image or the second ultrasound image so as to correspond with the one frame.

[12] A method of controlling an ultrasound diagnostic apparatus, the method comprising: acquiring first ultrasound images of a plurality of frames which are consecutive in time series and in which a first cross section of a heart of a subject is imaged, and second ultrasound images of a plurality of frames which are consecutive in time series and in which a second cross section of the heart different from the first cross section is imaged; calculating a cross-sectional area of a cavity of the heart for each frame for the first ultrasound images of the plurality of frames and the second ultrasound images of the plurality of frames; generating a first cross-sectional area change curve of the first cross section and a second cross-sectional area change curve of the second cross section, each of which represents a temporal change in the cross-sectional area, based on the calculated cross-sectional area; aligning the first cross-sectional area change curve and the second cross-sectional area change curve, which are generated, with each other; displaying the first ultrasound image and the second ultrasound image at corresponding points on the first cross-sectional area change curve and the second cross-sectional area change curve; and automatically updating, in a case in which one frame of the first ultrasound image or the second ultrasound image displayed on the monitor is updated based on an instruction from a user, the other frame of the first ultrasound image or the second ultrasound image so as to correspond with the one frame.

Since the ultrasound diagnostic apparatus according to the aspect of the present invention comprises: the image acquisition unit that acquires the first ultrasound images of the plurality of frames which are consecutive in time series and in which the first cross section of the heart of the subject is imaged, and the second ultrasound images of the plurality of frames which are consecutive in time series and in which the second cross section of the heart different from the first cross section is imaged; the cross-sectional area calculation unit that calculates the cross-sectional area of the cavity of the heart for each frame for the first ultrasound images of the plurality of frames and the second ultrasound images of the plurality of frames; the cross-sectional area change curve generation unit that generates the first cross-sectional area change curve of the first cross section and the second cross-sectional area change curve of the second cross section, each of which represents the temporal change in the cross-sectional area, based on the cross-sectional area calculated by the cross-sectional area calculation unit; the alignment unit that aligns the first cross-sectional area change curve and the second cross-sectional area change curve, which are generated by the cross-sectional area change curve generation unit, with each other; the monitor that displays the first ultrasound image and the second ultrasound image at the corresponding points on the first cross-sectional area change curve and the second cross-sectional area change curve; and the frame update unit that, in a case in which one frame of the first ultrasound image or the second ultrasound image displayed on the monitor is updated based on the instruction from the user, updates the other frame of the first ultrasound image or the second ultrasound image so as to correspond with the one frame, it is possible to easily calculate the ejection fraction of the cavity of the heart in a short time.

## BRIEF DESCRIPTION OF THE DRAWINGS

Fig. 1 is a block diagram showing a configuration of an ultrasound diagnostic apparatus according to Embodiment 1 of the present invention.

Fig. 2 is a block diagram showing an internal configuration of a transmission-and-reception circuit in Embodiment 1 of the present invention.

Fig. 3 is a block diagram showing an internal configuration of an image generation unit in Embodiment 1 of the present invention.

Fig. 4 is a schematic diagram of an example of a first ultrasound image representing an apical four-chamber cross section.

Fig. 5 is a schematic diagram of an example of a second ultrasound image representing an apical two-chamber cross section.

Fig. 6 is a schematic diagram of an example of a first cross-sectional area change curve representing a temporal change in a cross-sectional area of a cavity of a heart in the first ultrasound images of a plurality of frames.

Fig. 7 is a schematic diagram of an example of a second cross-sectional area change curve representing a temporal change in a cross-sectional area of the cavity of the heart in the second ultrasound images of a plurality of frames.

Fig. 8 is a block diagram showing an internal configuration of an alignment unit in Embodiment 1 of the present invention.

Fig. 9 is a diagram showing a first curved portion selected from the first cross-sectional area change curve and a second curved portion selected from the second cross-sectional area change curve.

Fig. 10 is a schematic diagram of a two-dimensional array of values of points on a first curve and values of points on a second curve in Embodiment 1.

Fig. 11 is a schematic diagram of display examples of the first ultrasound image, the second ultrasound image, the first cross-sectional area change curve, and the second cross-sectional area change curve.

Fig. 12 is a flowchart showing an operation of the ultrasound diagnostic apparatus according to Embodiment 1 of the present invention.

Fig. 13 is a flowchart showing alignment processing in Embodiment 1 of the present invention.

Fig. 14 is a block diagram showing a configuration of an ultrasound diagnostic apparatus according to Embodiment 2 of the present invention.

Fig. 15 is a block diagram showing an internal configuration of an alignment unit in Embodiment 2 of the present invention.

Fig. 16 is a diagram showing a first cross-sectional area change curve and a similar portion of a second cross-sectional area change curve similar to the first cross-sectional area change curve.

Fig. 17 is a schematic diagram of a two-dimensional array of values of points on a first curve and values of points on a second curve in Embodiment 2.

Fig. 18 is a flowchart showing alignment processing in Embodiment 2 of the present invention.

## DESCRIPTION OF THE PREFERRED EMBODIMENTS

Hereinafter, embodiments of the present invention will be described with reference to the accompanying drawings.

The following configuration requirements are described based on a representative embodiment of the present invention, but the present invention is not limited to such an embodiment.

The present specification, a numerical range represented by "to" means a range including numerical values described before and after "to", both ends inclusive, as a lower limit value and an upper limit value.

In the present specification, "the same" includes an error range generally allowed in the technical field.

Embodiment 1

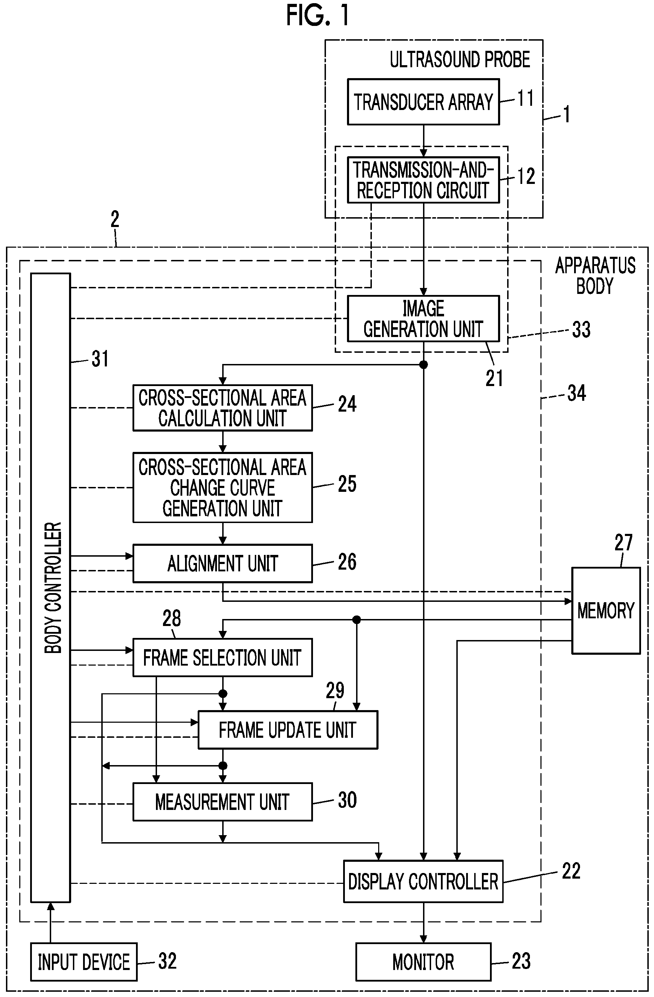

Fig. 1 shows a configuration of an ultrasound diagnostic apparatus according to Embodiment 1 of the present invention. The ultrasound diagnostic apparatus comprises an ultrasound probe 1 and an apparatus body 2 that are connected to each other by so-called wired communication or so-called wireless communication.

The ultrasound probe 1 comprises a transducer array 11 and a transmission-and-reception circuit 12 connected to the transducer array 11.

The apparatus body 2 comprises an image generation unit 21 connected to the transmission-and-reception circuit 12. In the apparatus body 2, a display controller 22 and a monitor 23 are sequentially connected to the image generation unit 21. Further, a cross-sectional area calculation unit 24 is connected to the image generation unit 21. A cross-sectional area change curve generation unit 25 and an alignment unit 26 are sequentially connected to the cross-sectional area calculation unit 24. A memory 27 is connected to the alignment unit 26. A frame selection unit 28 is connected to the memory 27. A frame update unit 29 is connected to the memory 27 and the frame selection unit 28. A measurement unit 30 is connected to the frame selection unit 28 and the frame update unit 29. The memory 27, the frame selection unit 28, the frame update unit 29, and the measurement unit 30 are each connected to the display controller 22. In addition, a body controller 31 is connected to the transmission-and-reception circuit 12, the image generation unit 21, the display controller 22, the cross-sectional area calculation unit 24, the cross-sectional area change curve generation unit 25, the alignment unit 26, the memory 27, the frame selection unit 28, the frame update unit 29, and the measurement unit 30. An input device 32 is connected to the body controller 31.

The transmission-and-reception circuit 12 and the image generation unit 21 constitute an image acquisition unit 33. In addition, the image generation unit 21, the display controller 22, the cross-sectional area calculation unit 24, the cross-sectional area change curve generation unit 25, the alignment unit 26, the frame selection unit 28, the frame update unit 29, the measurement unit 30, and the body controller 31 constitute a processor 34 for the apparatus body 2.

The transducer array 11 of the ultrasound probe 1 includes a plurality of ultrasound transducers that are arranged one-dimensionally or two-dimensionally. In accordance with a drive signal supplied from the transmission-and-reception circuit 12, each of the ultrasound transducers transmits ultrasound and receives an ultrasound echo from a subject to output a signal based on the ultrasound echo. Each ultrasound transducer is configured by, for example, forming electrodes at both ends of a piezoelectric material consisting of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), and the like.

The image acquisition unit 33 configured by the transmission-and-reception circuit 12 and the image generation unit 21 acquires an ultrasound image of an inside of the subject by transmitting and receiving an ultrasound beam by using the ultrasound probe 1.

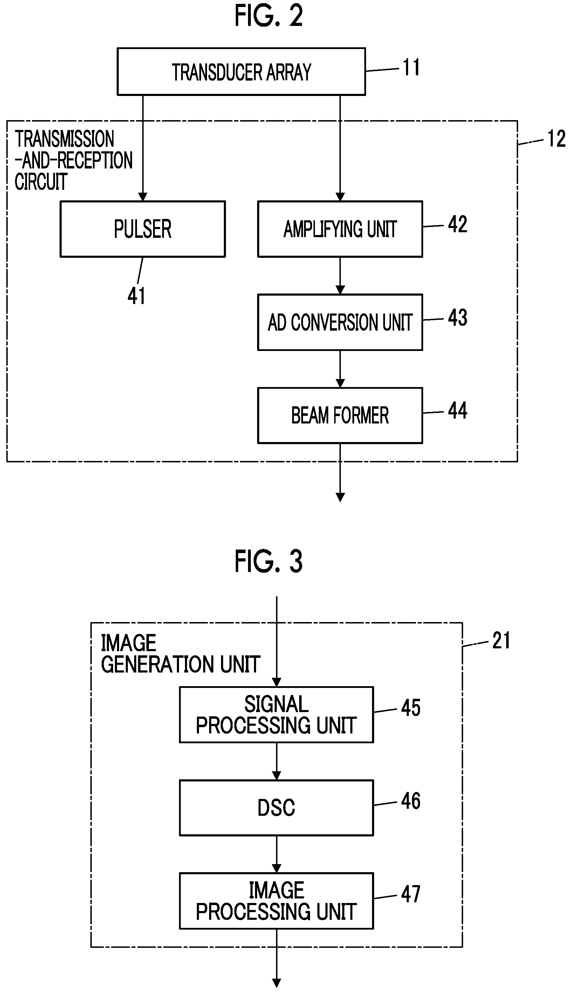

The transmission-and-reception circuit 12 transmits the ultrasound wave from the transducer array 11 and generates a sound ray signal based on the reception signal acquired by the transducer array 11, under the control of the body controller 31. As shown in Fig. 2, the transmission-and-reception circuit 12 includes a pulser 41 connected to the transducer array 11, and an amplifying unit 42, an analog-to-digital (AD) conversion unit 43, and a beam former 44 that are sequentially connected in series to the transducer array 11.

The pulser 41 includes, for example, a plurality of pulse generators, and the pulser 41 adjusts an amount of delay of each drive signal such that the ultrasound transmitted from the plurality of ultrasound transducers of the transducer array 11 form the ultrasound beam, based on a transmission delay pattern selected in accordance with a control signal from the body controller 31, and supplies the adjusted signal to the plurality of ultrasound transducers. In this way, in a case in which a pulsed or continuous wave-like voltage is applied to the electrodes of the ultrasound transducer of the transducer array 11, the piezoelectric material expands and contracts to generate pulsed or continuous wave-like ultrasound from each of the ultrasound transducers, and then the ultrasound beam is formed from the combined wave of the ultrasound.

The transmitted ultrasound beam is, for example, reflected by a target such as a part of the subject and propagates toward the transducer array 11 of the ultrasound probe 1. The ultrasound echo propagating toward the transducer array 11 as described above is received by each of the ultrasound transducers constituting the transducer array 11. In this case, each of the ultrasound transducers constituting the transducer array 11 receives the propagating ultrasound echo to expand and contract, generates the reception signal, which is an electrical signal, and outputs these reception signals to the amplifying unit 42.

The amplifying unit 42 amplifies the signal input from each of the ultrasound transducers constituting the transducer array 11, and transmits the amplified signal to the AD conversion unit 43. The AD conversion unit 43 converts the signal, which is transmitted from the amplifying unit 42, into digital reception data. The beam former 44 performs so-called reception focus processing by applying respective delays to the reception data received from the AD conversion unit 43 and then adding the delayed data together. By this reception focus processing, each reception data, which is converted by the AD conversion unit 43, is phase-added, and the sound ray signal in which the focus of the ultrasound echo is narrowed down is acquired.

As shown in Fig. 3, the image generation unit 21 has a configuration in which a signal processing unit 45, a digital scan converter (DSC) 46, and an image processing unit 47 are sequentially connected in series.

The signal processing unit 45 generates a B-mode image signal, which is tomographic image information related to tissues inside the subject, by performing, on the sound ray signal received from the transmission-and-reception circuit 12, correction of the attenuation due to a distance in accordance with a depth of a reflection position of the ultrasound by using a sound velocity value set by the body controller 31 and then performing envelope detection processing.

The DSC 46 converts (raster-converts) the B-mode image signal, which is generated by the signal processing unit 45, into the image signal in accordance with a normal television signal scanning method.

The image processing unit 47 performs various types of necessary image processing such as gradation processing on the B-mode image signal input from the DSC 46, and then sends the B-mode image signal to the display controller 22 and the cross-sectional area calculation unit 24. Hereinafter, the B-mode image signal, which is image-processed by the image processing unit 47, will be referred to as an ultrasound image.

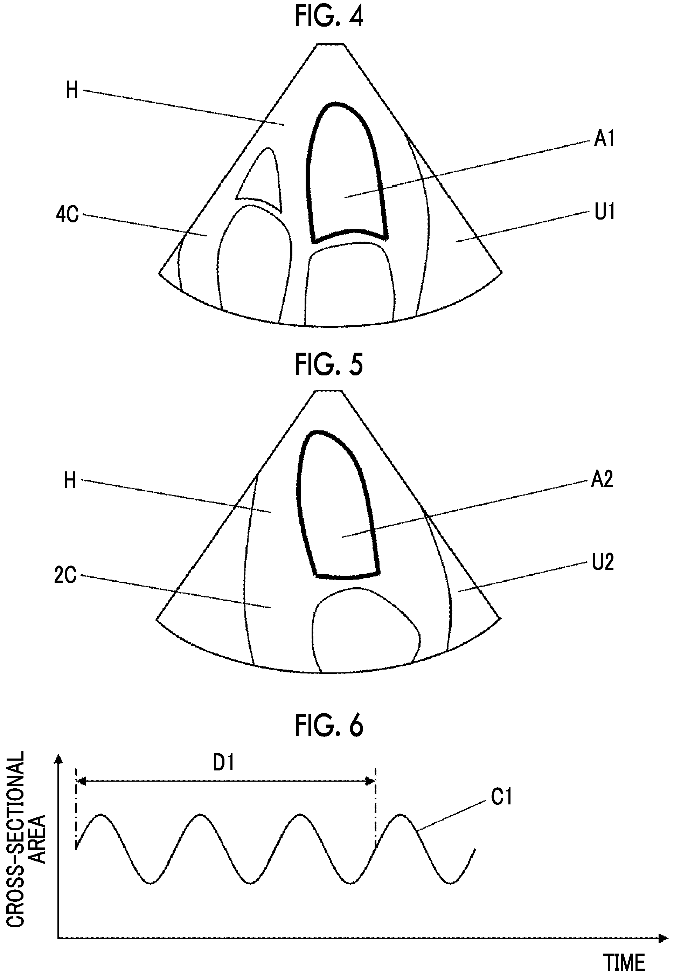

The ultrasound diagnostic apparatus according to Embodiment 1 of the present invention is used to calculate an ejection fraction of a cavity of a heart of a subject, such as a left ventricular ejection fraction (LVEF). In order to calculate the ejection fraction of the cavity of the heart, the image acquisition unit 33 acquires first ultrasound images U1 of a plurality of frames representing a so-called apical four-chamber cross section 4C (first cross section) of the heart H and second ultrasound images U2 of a plurality of frames representing a so-called apical two-chamber cross section 2C (second cross section) of the heart H, as shown in Fig. 4.

The display controller 22 performs predetermined processing on the first ultrasound image U1, the second ultrasound image U2, and the like generated by the image generation unit 21, and displays the first ultrasound image U1, the second ultrasound image U2, and the like on the monitor 23 under the control of the body controller 31.

The monitor 23 displays the first ultrasound image U1, the second ultrasound image U2, and the like under the control of the display controller 14, and includes, for example, a display device such as a liquid crystal display (LCD) or an organic electroluminescence display (organic EL display).

The cross-sectional area calculation unit 24 calculates a cross-sectional area of the cavity of the heart H for each frame for the first ultrasound images U1 of the plurality of frames and the second ultrasound images U2 of the plurality of frames. The cross-sectional area of the cavity of the heart H calculated here refers to an area of the cavity of the heart H on the first ultrasound image U1 and an area of the cavity of the heart H on the second ultrasound image U2. For example, in a case in which the purpose is to calculate the left ventricular ejection fraction, the cross-sectional area calculation unit 24 calculates a cross-sectional area of a cavity of a left ventricle A1 in the first ultrasound images U1 of the plurality of frames as shown in Fig. 4 and a cross-sectional area of a cavity of a left ventricle A2 in the second ultrasound images U2 of the plurality of frames as shown in Fig. 5.

The cross-sectional area calculation unit 24 can input the first ultrasound image U1 and the second ultrasound image U2 to so-called a trained model in machine learning, which has learned a relationship between a plurality of ultrasound images and the cavity of the heart included in the ultrasound images and the area of the cavity, to output the cross-sectional area of the cavity of the heart H in the first ultrasound image U1 and the cross-sectional area of the cavity of the heart H in the second ultrasound image U2.

In addition, the cross-sectional area calculation unit 24 can extract the cavity of the heart H from each of the first ultrasound image U1 and the second ultrasound image U2 by using a so-called template matching method of, for example, storing a plurality of template image data representing the cavity of the heart H in advance and searching for the first ultrasound image U1 and the second ultrasound image U2 using the plurality of template image data, and calculate the cross-sectional area of the obtained cavity of the heart H. As described above, the cross-sectional area calculation unit 24 can extract a contour of the cavity of the heart H, an image region occupied by the cavity of the heart H, or the like from the image by using a known algorithm, and calculate the cross-sectional area of the cavity of the heart H based on the extracted contour, image region, or the like of the cavity of the heart H.

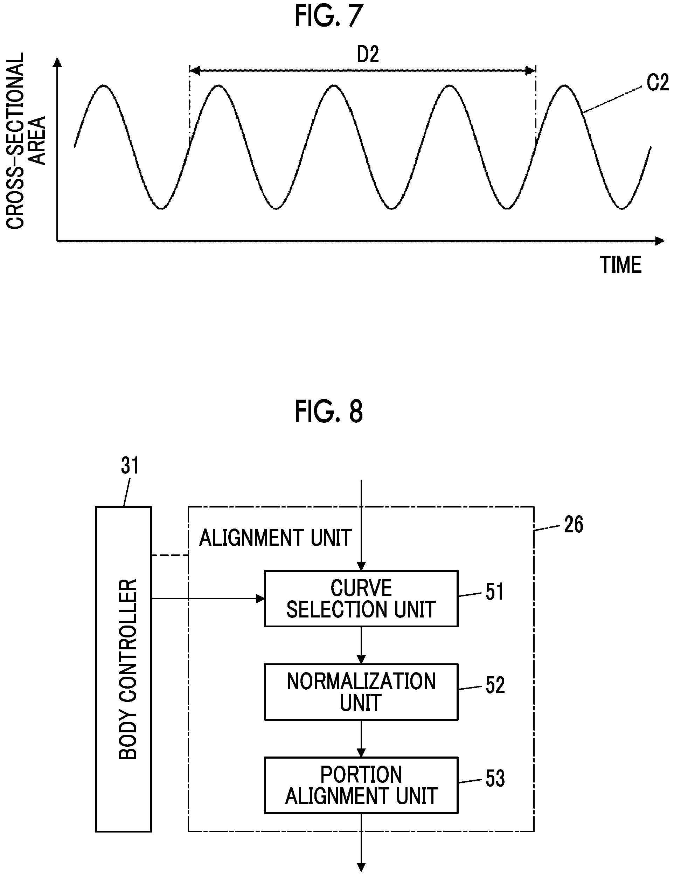

The cross-sectional area change curve generation unit 25 generates a first cross-sectional area change curve of apical four-chamber cross section 4C and a second cross-sectional area change curve of the apical two-chamber cross section 2C, each of which represents a temporal change in the cross-sectional area, based on the cross-sectional area of the cavity of the heart H calculated by the cross-sectional area calculation unit 24. Fig. 6 shows an example of a first cross-sectional area change curve C1, and Fig. 7 shows an example of a second cross-sectional area change curve C2. It is known that the cross-sectional area of the cavity of the heart H in the apical four-chamber cross section 4C and the apical two-chamber cross section 2C periodically changes in time series due to the beating of the heart H. In addition, a heart rate of the subject is not always constant due to a disease such as arrhythmia of the subject, a mental or physical burden on the subject, or the like, and imaging conditions for the apical four-chamber cross section 4C and the apical two-chamber cross section 2C may also be different. Therefore, in the first cross-sectional area change curve C1 and the second cross-sectional area change curve C2, the period in which the cross-sectional area of the cavity of the heart H changes and the scale of the cross-sectional area may be different from each other.

The alignment unit 26 aligns the first cross-sectional area change curve C1 and the second cross-sectional area change curve C2 with each other. Here, the alignment between the first cross-sectional area change curve C1 and the second cross-sectional area change curve C2 refers to associating a maximal value in the first cross-sectional area change curve C1 and a maximal value in the second cross-sectional area change curve C2, a minimal value in the first cross-sectional area change curve C1 and a minimal value in the second cross-sectional area change curve C2, and each point between the maximal value and the minimal value in the first cross-sectional area change curve C1 and each point between the maximal value and the minimal value in the second cross-sectional area change curve C2 with each other.

As shown in Fig. 8, the alignment unit 26 has a configuration in which a curve selection unit 51, a normalization unit 52, and a portion alignment unit 53 are connected in series.

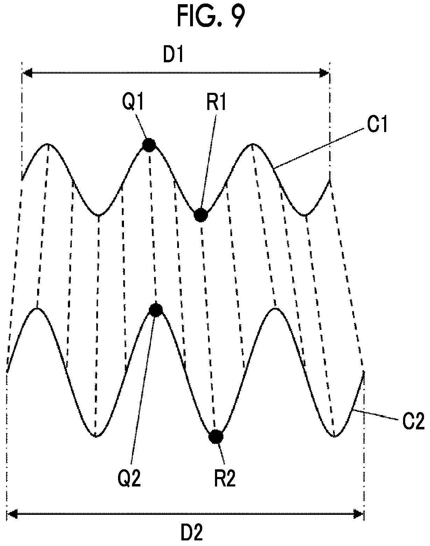

For example, as shown in Fig. 9, the curve selection unit 51 selects a first curved portion D1 and a second curved portion D2, which include the same number of change cycles, from the first cross-sectional area change curve C1 and the second cross-sectional area change curve C2. The change cycle represents a portion corresponding to one period of the change in the cross-sectional area in the first cross-sectional area change curve C1 and the second cross-sectional area change curve C2, and the end points of the period can be optionally set.

The curve selection unit 51 can select the first curved portion D1 and the second curved portion D2 based on sections selected by the user via the input device 32 for, for example, the first cross-sectional area change curve C1 and the second cross-sectional area change curve C2. For example, in a case in which the input device 32 includes a mouse, a touch panel, or the like, the user can select the sections in the first cross-sectional area change curve C1 and the second cross-sectional area change curve C2 by a so-called drag operation, and in a case in which the input device 32 includes a so-called keyboard or the like, the user can select the sections by inputting a start point in time and an end point in time of the sections in the first cross-sectional area change curve C1 and the second cross-sectional area change curve C2.

In addition, the curve selection unit 51 can select the first curved portion D1 or the second curved portion D2 based on the section selected by the user in the first cross-sectional area change curve C1 or the second cross-sectional area change curve C2, calculate the number of change cycles included in one of the first curved portion D1 or the second curved portion D2, and select the other of the first curved portion D1 or the second curved portion D2 based on the calculated number of change cycles. For example, in a case in which the first curved portion D1 including three change cycles is selected from the first cross-sectional area change curve C1 by the user, the curve selection unit 51 can select the second curved portion D2 that is located at the central portion of the second cross-sectional area change curve C2 and includes the same three change cycles as the first curved portion D1. In this case, the position on the second cross-sectional area change curve C2 where the curve selection unit 51 selects the second curved portion D2 can be set in advance, such as a central portion or an end portion.

In addition, the curve selection unit 51 can calculate the number of change cycles included in the entire first cross-sectional area change curve C1 and the number of change cycles included in the entire second cross-sectional area change curve C2, and select the first curved portion D1 and the second curved portion D2 which include the same number of change cycles as a designated number of cycles designated by the user via the input device 32 by referring to the calculated number of change cycles. The position on the first cross-sectional area change curve C1 where the curve selection unit 51 selects the first curved portion D1 and the position on the second cross-sectional area change curve C2 where the curve selection unit 51 selects the second curved portion D2 in this case can be set in advance, such as the central portion or the end portion.

The normalization unit 52 normalizes the first curved portion D1 and the second curved portion D2, which are selected by the curve selection unit 51, using the respective maximum values thereof. Normalizing the first curved portion D1 using the maximum value means dividing all values of the first curved portion D1 by the maximum value in the first curved portion D1, and normalizing the second curved portion D2 using the maximum value means dividing all values of the second curved portion D2 by the maximum value in the second curved portion D2. Each point on the normalized first curved portion D1 and each point on the normalized second curved portion D2 have values greater than 0 and equal to or less than 1.

The portion alignment unit 53 aligns the first curved portion D1 and the second curved portion D2, which are selected by the curve selection unit 51, with each other. The portion alignment unit 53 can align the first curved portion D1 and the second curved portion D2 with each other using, for example, an algorithm such as a so-called dynamic time warping (DTW).

Specifically, the portion alignment unit 53 divides a section from the start point in time to the end point in time of the first curved portion D1 into M parts at regular time intervals and sets a first matrix X representing the normalized values in the first curved portion D1 corresponding to the M divided points in time as in Expression (1). Here, M is a positive integer equal to or greater than 2, but it is preferable to set M to a number as large as possible in order to perform accurate alignment.

$$X = (X1, X2, \cdots, XM) \quad \bullet \bullet \bullet \; (1)$$

In addition, the portion alignment unit 53 divides a section from the start point in time to the end point in time of the second curved portion D2 into N parts with the same time interval as the time interval used for dividing the section from the start point in time to the end point in time of the first curved portion D1, and sets a second matrix Y representing the normalized values in the second curved portion D2 corresponding to the N divided points in time as in Expression (2). Here, N is a positive integer equal to or greater than 2, but it is preferable to set N to a number as large as possible in order to perform accurate alignment.

$$Y = (Y1, Y2, \cdots, YN) \quad \bullet \bullet \bullet \; (2)$$

The portion alignment unit 53 sets a difference matrix D representing a difference in all combinations of each element of the first matrix X and each element of the second matrix Y as in Expression (3).

$$D = \begin{pmatrix} |X1 - Y1| & |X1 - Y2| & \cdots & |X1 - YN| \\ |X2 - Y1| & |X2 - Y2| & \cdots & |X2 - YN| \\ \vdots & \vdots & \ddots & \vdots \\ |XM - Y1| & |XM - Y2| & \cdots & |XM - YN| \end{pmatrix} \bullet \bullet \bullet \; (3)$$

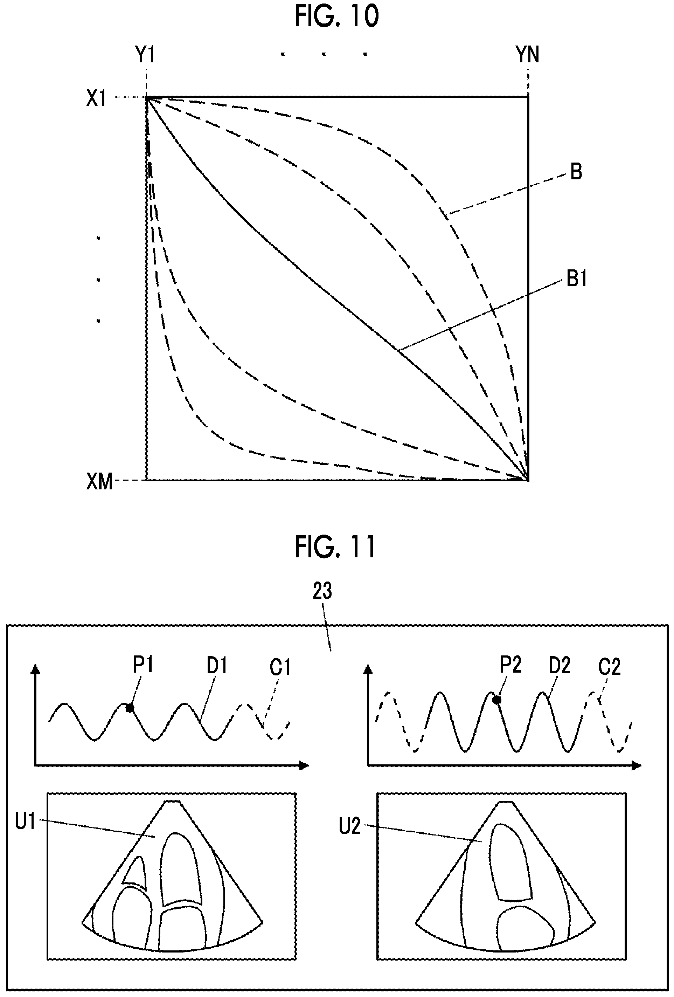

The portion alignment unit 53 calculates, using an algorithm such as a so-called dynamic programming, as schematically shown in Fig. 10, a shortest route B1 in which a sum of all elements on the route is minimized, the shortest route B1 being a route that is continuous and does not return to |X1 - Y1| among a plurality of routes B from |X1 - Y1| to |XM - YN| in a plurality of elements in the difference matrix D. Fig. 10 is a schematic diagram of a two-dimensional array of each element of X represented by Expression (1) and each element of Y represented by Expression (2), which corresponds to the difference matrix D represented by Expression (3), and a point (XL, YK) on the two-dimensional array corresponds to an element |XL - YK| in the difference matrix D. Here, L is an integer of equal to or greater than 1 and equal to or less than M, and K is an integer of equal to or greater than 1 and equal to or less than N.

The portion alignment unit 53 can aligns the points corresponding to the M divided points in time on the first curved portion D1 and the points corresponding to the M divided points in time among the N divided points in time on the second curved portion D2 as shown in Fig. 9 by associating XL and YK used to calculate the difference in each element |XL - YK| on the shortest route B1 in the difference matrix D with each other. It should be noted that the points, corresponding to the M divided points in time, on the second curved portion D2 include a point corresponding to the element Y1 of the second matrix Y and a point corresponding to the element YN.

The memory 27 stores the first curved portion D1 and the second curved portion D2 aligned by the alignment unit 26 under the control of the body controller 31. In addition, under the control of the body controller 31, the memory 27 reads out the stored first curved portion D1 and second curved portion D2 and sends the first curved portion D1 and the second curved portion D2 to the display controller 22, the frame selection unit 28, and the frame update unit 29.

In addition, as the memory 27, for example, a recording medium such as a flash memory, a hard disk drive (HDD), a solid-state drive (SSD), a flexible disk (FD), a magneto-optical disk (MO disk), a magnetic tape (MT), a random-access memory (RAM), a compact disc (CD), a digital versatile disc (DVD), a secure digital card (SD card), or a universal serial bus memory (USB memory) can be used.

As shown in Fig. 9, the frame selection unit 28 selects, as a first measurement frame group, one set of first ultrasound images U1 corresponding to a maximal value Q1 and a minimal value R1 in the first curved portion D1 of the first cross-sectional area change curve C1. In addition, the frame selection unit 28 selects, as a second measurement frame group, one set of second ultrasound images U2 corresponding to a maximal value Q2 and a minimal value R2 in the second cross-sectional area change curve C2, which are aligned with the maximal value Q1 and the minimal value R1 in the first cross-sectional area change curve C1 by the alignment unit 26.

The frame selection unit 28 can select a first frame group for the first curved portion D1 based on, for example, the instruction from the user via the input device 32. The frame selection unit 28 can automatically select a second frame group corresponding to the first frame group selected based on the instruction from the user from a correspondence relationship between the first curved portion D1 and the second curved portion D2 obtained by the alignment unit 26.

Further, the frame selection unit 28 can automatically extract a plurality of maximal values Q1 and a plurality of minimal values R1 in the first curved portion D1, and can select one set of the maximal value Q1 and the minimal value R1 from among the plurality of extracted maximal values Q1 and the plurality of extracted minimal values R1. In this case, for example, the frame selection unit 28 can select the largest maximal value Q1 among the plurality of maximal values Q1 and select the smallest minimal value R1 among the plurality of minimal values R1.

As shown in Fig. 11, the monitor 23 can display the first ultrasound image U1 and the second ultrasound image U2 at the corresponding points P1 and P2 on the first cross-sectional area change curve C1 and the second cross-sectional area change curve C2, for example, the first ultrasound image U1 of the first frame group and the second ultrasound image U2 of the second frame group, which are selected by the frame selection unit 28. In the example of Fig. 11, the first cross-sectional area change curve C1, the first curved portion D1, the point P1 on the first curved portion D1, the second cross-sectional area change curve C2, the second curved portion D2, the point P2 on the second curved portion D2, the first ultrasound image U1, and the second ultrasound image U2 are displayed on the monitor 23.

In a case in which one frame of the first ultrasound image U1 or the second ultrasound image U2 displayed on the monitor 23 is updated based on the instruction from the user, the frame update unit 29 updates the other frame of the first ultrasound image U1 or the second ultrasound image U2 corresponding to the one frame by referring to the correspondence relationship between the first curved portion D1 and the second curved portion D2 aligned by the alignment unit 26.

For example, in a case in which the user checks the first ultrasound image U1 of the first frame group and the second ultrasound image U2 of the second frame group selected by the frame selection unit 28 and displayed on the monitor 23 and determines that the first ultrasound image U1 of the first frame group and the second ultrasound image U2 of the second frame group are not suitable for calculating the ejection fraction of the cavity of the heart H, the user can issue an instruction to update one frame of the first ultrasound image U1 of the first frame group or the second ultrasound image U2 of the second frame group, for example, an instruction to update the first ultrasound image U1 to the first ultrasound image U1 of one frame before. In this case, the frame update unit 29 automatically updates the second ultrasound image U2, which is displayed on the monitor 23, to the second ultrasound image U2 corresponding to the updated first ultrasound image U1.

As described above, in a case in which the user issues the instruction to update one of the first ultrasound image U1 or the second ultrasound image U2, the other is also automatically updated, so that the user can easily find both the first ultrasound image U1 and the second ultrasound image U2 suitable for measuring the ejection fraction of the cavity of the heart H in a short time.

The measurement unit 30 calculates the ejection fraction of the cavity of the heart H, such as the left ventricular ejection fraction, from the cross-sectional area of the cavity of the heart H corresponding to each of the first frame group and the second frame group selected by the frame selection unit 28 or the cross-sectional area of the cavity of the heart H corresponding to each of the first frame group and the second frame group updated by reflecting the instruction from the user by the frame update unit 29. The measurement unit 30 calculates the ejection fraction of the cavity of the heart H from the cross-sectional area of the cavity of the heart H corresponding to each of the first frame group and the second frame group selected by the frame selection unit 28 in a case in which there is no update of the first frame group and the second frame group by the frame update unit 29, and calculates the ejection fraction of the cavity of the heart H from the cross-sectional area of the cavity of the heart H corresponding to each of the first frame group and the second frame group updated by the frame update unit 29 in a case in which there is the update of the first frame group and the second frame group by the frame update unit 29. Although not shown, the value of the ejection fraction of the cavity of the heart H measured by the measurement unit 30 can be displayed on the monitor 23 together with the first cross-sectional area change curve C1, the second cross-sectional area change curve C2, the first ultrasound image U1, and the second ultrasound image U2.

The body controller 31 controls each unit of the apparatus body 2 and the transmission-and-reception circuit 12 of the ultrasound probe 1 based on a control program and the like, which are stored in advance.

The input device 32 is an input device used by the user to perform an input operation and is configured by, for example, a device such as a keyboard, a mouse, a trackball, a touchpad, and a touch sensor disposed in a state of being superimposed on the monitor 23.

The processor 34 including the image generation unit 21, the display controller 22, the cross-sectional area calculation unit 24, the cross-sectional area change curve generation unit 25, the alignment unit 26, the frame selection unit 28, the frame update unit 29, the measurement unit 30, and the body controller 31 may be configured by one or a plurality of types of hardware, and the types of hardware are not limited. For example, the processor can be configured by a programmable logic device such as a central processing unit (CPU), a micro processing unit (MPU), or a field programmable gate array (FPGA), a dedicated circuit for executing specific processing, such as an application specific integrated circuit (ASIC), or hardware such as a graphic processing unit (GPU) or a neural processing unit (NPU). In addition, the processor includes each unit or each means that executes various types of processing in the present embodiment. Further, the types of hardware may be a combination of different types of hardware. In a case in which a plurality of types of hardware are configured to execute one or a plurality of types of processing of a certain processor, the plurality of types of hardware may be present in devices physically separated from each other or may be present in the same device. In addition, in any of the embodiments, the order of each processing performed by the processor is not limited to the above-described order, and may be changed as appropriate. In addition, hardware is implemented in a form of an electric circuit (circuitry) in which circuit elements such as semiconductor elements are combined.

Further, the present embodiment may be implemented by hardware, software, firmware, microcode, or a combination thereof. Software, firmware, and microcode are is implemented in a form of programs. In addition, the program may be, for example, a program module group, and each function thereof may be implemented by a processor configured to execute each function. The program may be a program code or a plurality of code segments stored in one or a plurality of non-transitory computer-readable media (for example, a storage medium or other storages). The program may be stored in a plurality of non-transitory computer-readable media present in devices physically separated from each other. The program code or the code segment can represent any combination of a procedure, a function, a subprogram, a routine, a subroutine, a module, a software package, a class, or an instruction, a data structure, or a program statement. The program code or the code segment may be connected to another code segment or a hardware circuit by transmitting and receiving information, data, an argument, a parameter, or a content of the memory.

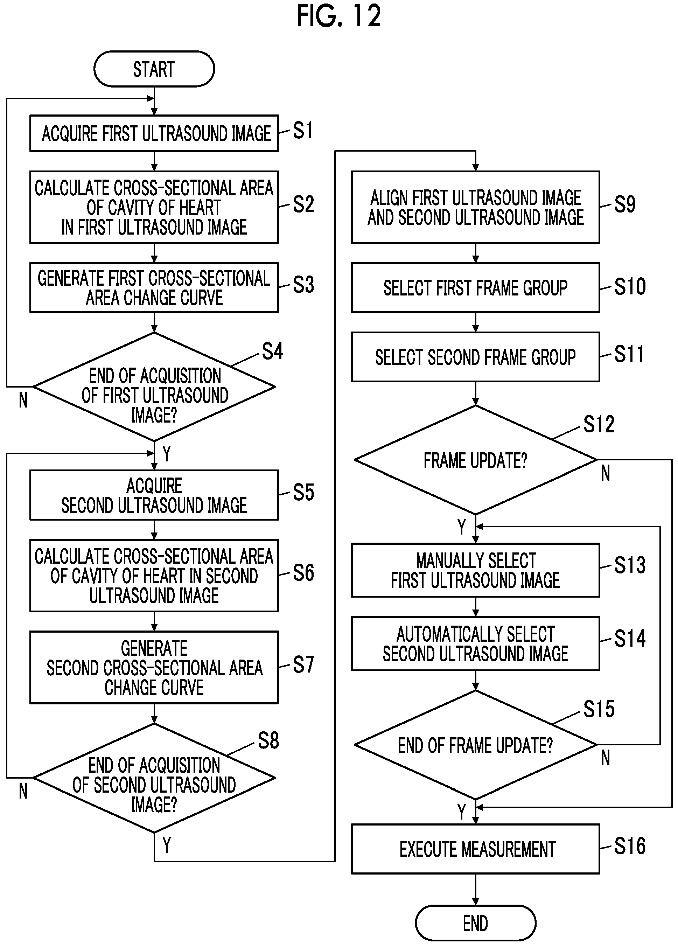

Hereinafter, an operation of the ultrasound diagnostic apparatus according to Embodiment 1 will be described with reference to a flowchart shown in Fig. 12.

In step S1, the image acquisition unit 33 generates the first ultrasound image U1 representing the apical four-chamber cross section 4C. In this case, under the control of the body controller 31, the transmission and reception of the ultrasound from the plurality of transducers of the transducer array 11 are started in accordance with the drive signal from the pulser 41 of the transmission-and-reception circuit 12 of the ultrasound probe 1, the ultrasound echo from the subject is received by the plurality of transducers of the transducer array 11, and the reception signal as the analog signal is output to the amplifying unit 42, is amplified, and then is subjected to the AD conversion via the AD conversion unit 43 to acquire the reception data.

The reception focus processing is performed on the reception data by the beam former 44, and the sound ray signal generated by the reception focus processing is sent to the image generation unit 21 of the apparatus body 2, and the first ultrasound image U1 representing the apical four-chamber cross section 4C is generated by the image generation unit 21. In this case, the signal processing unit 45 of the image generation unit 21 performs the correction of the attenuation in accordance with the depth of the reflection position of the ultrasound and the envelope detection processing on the sound ray signal, the DSC 46 performs the conversion into the image signal in accordance with the normal television signal scanning method, and the image processing unit 47 performs various types of necessary image processing, such as gradation processing. The first ultrasound image U1 generated in step S1 in this manner is displayed on the monitor 23 via the display controller 22 and is sent to the cross-sectional area calculation unit 24.

In step S2, the cross-sectional area calculation unit 24 calculates the cross-sectional area of the cavity of the heart H shown in the first ultrasound image U1 by image-analyzing the first ultrasound image U1 acquired in step S1. The cross-sectional area calculation unit 24 can calculate the cross-sectional area of the cavity of the heart H, for example, by using a trained model in machine learning, which has learned a relationship between a large number of first ultrasound images U1 and the cross-sectional area of the cavity of the heart H shown in the first ultrasound images U1.

In step S3, the cross-sectional area change curve generation unit 25 generates the first cross-sectional area change curve C1 by plotting the value of the cross-sectional area calculated in step S2 along a time axis.

In step S4, the body controller 31 determines whether or not to end the acquisition of the first ultrasound image U1. The body controller 31 can determine to end the acquisition of the first ultrasound image U1, for example, in a case in which the user inputs an instruction to end the capturing of the first ultrasound image U1 via the input device 32, and can determine to continue the acquisition of the first ultrasound image U1 in a case in which there is no particular instruction from the user via the input device 32.

In a case in which the body controller 31 determines to continue the acquisition of the first ultrasound image U1 in step S4, the processing returns to step S1, and the first ultrasound image U1 of a new frame is acquired. In step S2, the cross-sectional area of the cavity of the heart H in the first ultrasound image U1 is calculated, and the value of the cross-sectional area newly calculated in step S3 is additionally plotted on the first cross-sectional area change curve C1 generated in previous step S3 along the time axis, and the first cross-sectional area change curve C1 is updated. As described above, the first cross-sectional area change curve C1 including a plurality of change cycles as shown in Fig. 6 is obtained by repeating the processing of step S1 to step S4.

In a case in which it is determined in step S4 to end the acquisition of the first ultrasound image U1, the processing proceeds to step S5. In order to capture the second ultrasound image U2 representing the apical two-chamber cross section 2C, for example, the user rotates the ultrasound probe 1 by 90 degrees about a direction in which the ultrasound probe 1 is pressed against the subject while the ultrasound probe 1 is fixed at a current position on the body surface of the subject.

In step S5, the image acquisition unit 33 acquires the second ultrasound image U2 representing the apical two-chamber cross section 2C using the same method as the method of acquiring the first ultrasound image U1 representing the apical four-chamber cross section 4C in step S1.

In step S6, the cross-sectional area calculation unit 24 calculates the cross-sectional area of the cavity of the heart H shown in the second ultrasound image U2 acquired in step S5 using the same method as the method of calculating the cross-sectional area of the cavity of the heart H shown in the first ultrasound image U1 in step S2.

In step S7, the cross-sectional area change curve generation unit 25 generates the second cross-sectional area change curve C2 by plotting the value of the cross-sectional area calculated in step S6 along a time axis.

In step S8, the body controller 31 determines whether or not to end the acquisition of the second ultrasound image U2 using the same method as the method of determining whether or not to end the acquisition of the first ultrasound image U1 in step S4.

In a case in which the body controller 31 determines to continue the acquisition of the second ultrasound image U2 in step S8, the processing returns to step S5, and the second ultrasound image U2 of a new frame is acquired. In step S6, the cross-sectional area of the cavity of the heart H in the second ultrasound image U2 is calculated, and the value of the cross-sectional area newly calculated in step S7 is additionally plotted on the second cross-sectional area change curve C2 generated in previous step S7 along the time axis, and the second cross-sectional area change curve C2 is updated. As described above, the second cross-sectional area change curve C2 including a plurality of change cycles as shown in Fig. 7 is obtained by repeating the processing of step S5 to step S8.

In a case in which it is determined in step S8 to end the acquisition of the second ultrasound image U2, the processing proceeds to step S9.

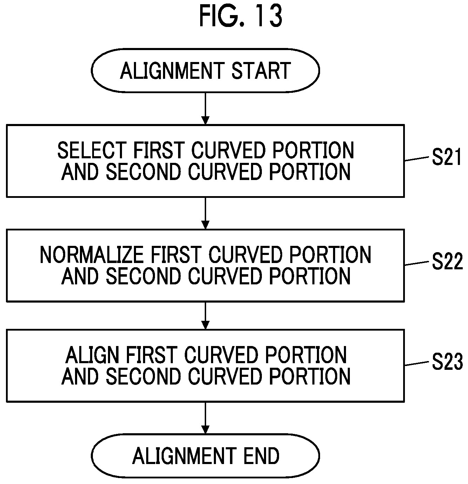

In step S9, the alignment unit 26 performs alignment between the first cross-sectional area change curve C1 generated in last step S3 and the second cross-sectional area change curve C2 generated in last step S7. Step S9 will be described in detail with reference to a flowchart of Fig. 13. As shown in this drawing, the processing of step S9 includes the processing of step S21 to step S23.

In step S21, the curve selection unit 51 of the alignment unit 26 selects, for example, the first curved portion D1 and the second curved portion D2 including the same number of change cycles from the first cross-sectional area change curve C1 and the second cross-sectional area change curve C2 as shown in Fig. 9. In this case, the curve selection unit 51 can select the first curved portion D1 corresponding to the section in the first cross-sectional area change curve C1 designated by the user via the input device 32 and the second curved portion D2 corresponding to the section in the second cross-sectional area change curve C2 designated by the user.

In step S22, the normalization unit 52 of the alignment unit 26 normalizes the first curved portion D1 and the second curved portion D2 selected in step S21 using the maximum values thereof.

In step S23, the portion alignment unit 53 of the alignment unit 26 performs alignment between the first curved portion D1 and the second curved portion D2 normalized in step S22 using an algorithm such as DTW. By performing the processing of step S23 in this way, the processing of the alignment in step S9 is completed. By performing steps S21 to S23, the first curved portion D1 and the second curved portion D2 can be accurately aligned by applying the algorithm such as DTW to the first curved portion D1 and the second curved portion D2, which include the same number of change cycles and are normalized.

In subsequent step S10, the frame selection unit 28 selects the first frame group consisting of the first ultrasound image U1 corresponding to the maximal value Q1 in the first curved portion D1 and the first ultrasound image U1 corresponding to the minimal value R1 from among the first ultrasound images U1 of the plurality of frames in order to obtain the first ultrasound image U1 of the frame suitable for calculating the ejection fraction of the cavity of the heart H. For example, the frame selection unit 28 can select a frame designated by the user via the input device 32 as the first ultrasound image U1 of the first frame group.

In step S11, the frame selection unit 28 automatically selects the second ultrasound images U2 of the second frame group corresponding to the first ultrasound images U1 of the first frame group selected in step S10 by referring to the correspondence relationship between the first curved portion D1 and the second curved portion D2 aligned with each other in step S9, in order to obtain the second ultrasound image U2 of the frame suitable for calculating the ejection fraction of the cavity of the heart H. Here, the second ultrasound image U2 of the second frame group refers to the second ultrasound image U2 corresponding to the maximal value Q2 in the second curved portion D2 and the second ultrasound image U2 corresponding to the minimal value R2.

As described above, the first ultrasound image U1 of the first frame group and the second ultrasound image U2 of the second frame group selected in step S10 and step S11 are displayed on the monitor 23 together with, for example, the first cross-sectional area change curve C1, the point P1 on the first cross-sectional area change curve C1 corresponding to the first ultrasound image U1 of the first frame group, the second cross-sectional area change curve C2, and the point P2 on the second cross-sectional area change curve C2 corresponding to the second ultrasound image U2 of the second frame group, as shown in Fig. 11.

In step S12, the frame update unit 29 determines whether or not to update the frames of the first ultrasound image U1 of the first frame group and the second ultrasound image U2 of the second frame group selected in step S10 and step S11. The frame update unit 29 can determine to update the frames of the first ultrasound image U1 of the first frame group and the second ultrasound image U2 of the second frame group in a case in which the user inputs the instruction to update the frames of the first ultrasound image U1 of the first frame group and the second ultrasound image U2 of the second frame group via the input device 32, for example, by the user determining that it is necessary to update the first ultrasound image U1 and the second ultrasound image U2 selected in step S10 and step S11 in order to accurately calculate the ejection fraction of the cavity of the heart H. In addition, for example, in a case in which the user does not input a specific instruction of whether or not to update the frames of the first ultrasound image U1 of the first frame group and the second ultrasound image U2 of the second frame group, the frame update unit 29 can determine not to update the frames of the first ultrasound image U1 of the first frame group and the second ultrasound image U2 of the second frame group and to maintain the frames.

In a case in which it is determined in step S12 to update the frames of the first ultrasound image U1 of the first frame group and the second ultrasound image U2 of the second frame group, the processing proceeds to step S13.

In step S13, the frame update unit 29 selects the first ultrasound image U1 of the first frame group based on the instruction from the user. In this case, the frame update unit 29 selects the first ultrasound image U1 of one frame designated by the user via the input device 32 from among the first ultrasound images U1 of the plurality of frames, for example, the first ultrasound image U1 of the frame adjacent to the first ultrasound image U1 selected in step S10.

In a case in which the first ultrasound image U1 is manually selected by the user in this way, the first ultrasound image U1 of the first frame group selected in step S10 is updated to the first ultrasound image U1 selected in step S13. As a result, the first ultrasound image U1, which is displayed on the monitor 23, is also updated to the first ultrasound image U1 selected in step S13.

In step S14, the frame update unit 29 automatically selects the second ultrasound image U2 corresponding to the first ultrasound image U1 of the first frame group selected in step S13 by referring to the correspondence relationship between the first curved portion D1 and the second curved portion D2 aligned in step S9.

In a case in which the second ultrasound image U2 is automatically selected in this way, the second ultrasound image U2 of the second frame group selected in step S11 is automatically updated to the second ultrasound image U2 selected in step S14. As a result, the second ultrasound image U2, which is displayed on the monitor 23, is also automatically updated to the second ultrasound image U2 selected in step S14.

In step S15, the frame update unit 29 determines whether or not to end the update of the frames of the first ultrasound image U1 of the first frame group and the second ultrasound image U2 of the second frame group. For example, the frame update unit 29 can determine to continue the update of the frames of the first ultrasound image U1 of the first frame group and the second ultrasound image U2 of the second frame group in a case in which the instruction to continue the update of the frames is input from the user via the input device 32, and determine to end the update of the frames of the first ultrasound image U1 of the first frame group and the second ultrasound image U2 of the second frame group in a case in which the instruction to end the update of the frames is input from the user via the input device 32.

In a case in which it is determined to continue the update of the frame in step S15, the processing returns to step S13, and the first ultrasound image U1 is manually selected by the user. In subsequent step S14, the second ultrasound image U2 corresponding to the first ultrasound image U1 selected in step S13 is automatically selected.

In the repetition of step S13 to step S15 performed in this manner, the user can select the first ultrasound image U1 of the first frame group and the second ultrasound image U2 of the second frame group, which are optimal for measuring the ejection fraction of the cavity of the heart H, while checking the first ultrasound image U1 of the first frame group and the second ultrasound image U2 of the second frame group displayed on the monitor 23 while being updated at any time. Further, since the second ultrasound image U2 corresponding to the first ultrasound image U1 is automatically selected and displayed on the monitor 23 only by the user selecting the first ultrasound image U1, the first ultrasound image U1 of the first frame group and the second ultrasound image U2 of the second frame group, which are optimal for measuring the ejection fraction of the cavity of the heart H, can be easily selected in a short time.

In a case in which it is determined in step S12 not to update the frames of the first ultrasound image U1 of the first frame group and the second ultrasound image U2 of the second frame group, and in a case in which it is determined in step S15 to end the update of the frames of the first ultrasound image U1 of the first frame group and the second ultrasound image U2 of the second frame group, the processing proceeds to step S16.

In a case in which it is determined that the frames of the first ultrasound image U1 of the first frame group and the second ultrasound image U2 of the second frame group are not updated in step S12, in step S16, the measurement unit 30 calculates the ejection fraction of the cavity of the heart H by using the value of the cross-sectional area of the cavity of the heart H corresponding to the first ultrasound image U1 of the first frame group selected in step S10 and the value of the cross-sectional area of the cavity of the heart H corresponding to the second ultrasound image U2 of the second frame group selected in step S11. In addition, in a case in which it is determined in step S15 to end the update of the frames of the first ultrasound image U1 of the first frame group and the second ultrasound image U2 of the second frame group, the measurement unit 30 calculates the ejection fraction of the cavity of the heart H by using the value of the cross-sectional area of the cavity of the heart H corresponding to the first ultrasound image U1 of the first frame group selected in step S13 and the value of the cross-sectional area of the cavity of the heart H corresponding to the second ultrasound image U2 of the second frame group selected in step S14.

The value of the ejection fraction of the cavity of the heart H calculated in step S16 can be displayed on the monitor 23 together with the first cross-sectional area change curve C1, the second cross-sectional area change curve C2, the first ultrasound image U1, and the second ultrasound image U2 as shown in Fig. 11. In a case in which the processing of step S16 is completed in this way, the operation of the ultrasound diagnostic apparatus according to Embodiment 1 shown in Fig. 12 is completed.

As described above, with the ultrasound diagnostic apparatus according to Embodiment 1, the cross-sectional area change curve generation unit 25 generates the first cross-sectional area change curve C1 and the second cross-sectional area change curve C2 based on the cross-sectional area calculated by the cross-sectional area calculation unit 24, the alignment unit 26 aligns the first cross-sectional area change curve C1 and the second cross-sectional area change curve C2 with each other, the monitor 23 displays the first ultrasound image U1 and the second ultrasound image U2 at the corresponding points on the first cross-sectional area change curve C1 and the second cross-sectional area change curve C2, and the frame update unit 29 automatically updates, in a case in which the one frame of the first ultrasound image U1 or the second ultrasound image U2 displayed on the monitor 23 is updated based on the instruction from the user, the other frame of the first ultrasound image U1 or the second ultrasound image U2 corresponding to the one frame, so that it is possible to easily calculate the ejection fraction of the cavity of the heart H in a short time.

In addition, a case has been described in which the transmission-and-reception circuit 12 is provided in the ultrasound probe 1, but the transmission-and-reception circuit 12 may be provided in the apparatus body 2.

A case has been described in which the image generation unit 21 is provided in the apparatus body 2, but the image generation unit 21 may be provided in the ultrasound probe 1.

The apparatus body 2 may be a so-called stationary type, a portable type that is easily carried, or a so-called handheld type that is configured by, for example, a smartphone or a tablet type computer. In this way, the type of the device constituting the apparatus body 2 is not particularly limited.

Although the apical four-chamber cross section 4C has been described as the first cross section corresponding to the first ultrasound image U1 and the apical two-chamber cross section 2C has been described as the second cross section corresponding to the second ultrasound image U2, the apical four-chamber cross section 4C can be treated as the second cross section and the apical two-chamber cross section 2C can be treated as the first cross section.

In a case in which the curve selection unit 51 of the alignment unit 26 selects the first curved portion D1 and the second curved portion D2 based only on the input from the user via the input device 32, the curve selection unit 51 can determine whether or not the number of change cycles included in the first curved portion D1 and the number of change cycles included in the second curved portion D2 are the same. In a case in which the number of change cycles included in the first curved portion D1 and the number of change cycles included in the second curved portion D2 are different from each other, the curve selection unit 51 can issue a warning to the user by, for example, displaying a message such as "Please select a curve to include the same number of change cycles" on the monitor 23.

The apparatus body 2 may have a cross-sectional area correction unit (not shown) that, after the first ultrasound image U1 of the first frame group and the second ultrasound image U2 of the second frame group used by the measurement unit 30 to calculate the ejection fraction of the cavity of the heart H are confirmed, corrects the contour of the cavity of the heart H or the image region occupied by the cavity of the heart H shown in these frames and corrects the cross-sectional area of the cavity of the heart H based on the corrected contour or image region. In this case, the cross-sectional area correction unit is, for example, connected to the frame selection unit 28, the frame update unit 29, the measurement unit 30, and the body controller 31.

The cross-sectional area correction unit corrects the contour of the cavity of the heart H or the image region occupied by the cavity of the heart H, which is shown in one of the first ultrasound image U1 of the first frame group or the second ultrasound image U2 of the second frame group, in accordance with the instruction from the user via the input device 32, and automatically corrects the contour of the cavity of the heart H or the image region occupied by the cavity of the heart H, which is shown in the other of the first ultrasound image U1 of the first frame group or the second ultrasound image U2 of the second frame group, by the same correction method as the correction of the cavity of the heart H by the user in the one frame. For example, in a case in which the correction of moving a part of the contour of the cavity of the heart H in the first ultrasound image U1 to a shallow portion is performed, the correction of moving the corresponding part of the contour of the cavity of the heart H in the second ultrasound image U2 to a shallow portion is automatically performed.

The cross-sectional area correction unit can output the second ultrasound image U2 corrected in the same manner as the first ultrasound image U1 by inputting the first ultrasound images U1 before and after the correction and the second ultrasound image U2 that has not been corrected to a trained model in machine learning, which has learned a large number of first ultrasound images U1 before and after the correction and a large number of second ultrasound images U2 before and after the correction.

Embodiment 2

The alignment unit 26 selects the first curved portion D1 and the second curved portion D2 from the first cross-sectional area change curve C1 and the second cross-sectional area change curve C2 based on the input from the user via the input device 32, but can also automatically select portions that are similar to each other in the first cross-sectional area change curve C1 and the second cross-sectional area change curve C2.

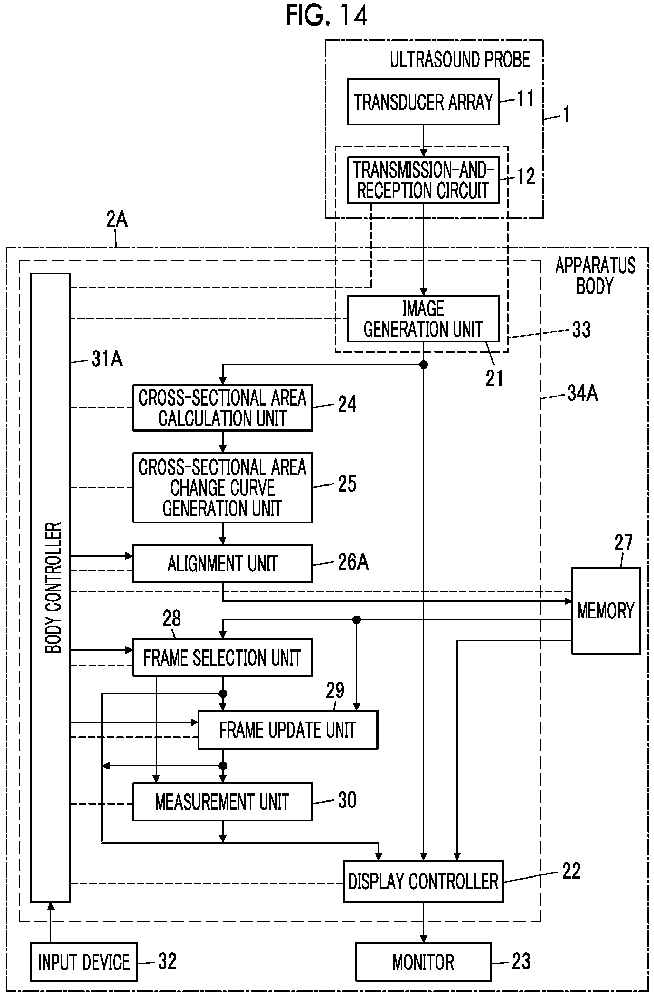

Fig. 14 is a block diagram of an ultrasound diagnostic apparatus according to Embodiment 2. The ultrasound diagnostic apparatus according to Embodiment 2 comprises an apparatus body 2A instead of the apparatus body 2 of the ultrasound diagnostic apparatus according to Embodiment 1 shown in Fig. 1. The apparatus body 2A according to Embodiment 2 comprises an alignment unit 26A instead of the alignment unit 26 of the apparatus body 2 according to Embodiment 1, and comprises a body controller 31A instead of the body controller 31 of the apparatus body 2 according to Embodiment 1. Further, the image generation unit 21, the display controller 22, the cross-sectional area calculation unit 24, the cross-sectional area change curve generation unit 25, the alignment unit 26A, the frame selection unit 28, the frame update unit 29, the measurement unit 30, and the body controller 31 constitute a processor 34A for the apparatus body 2A.

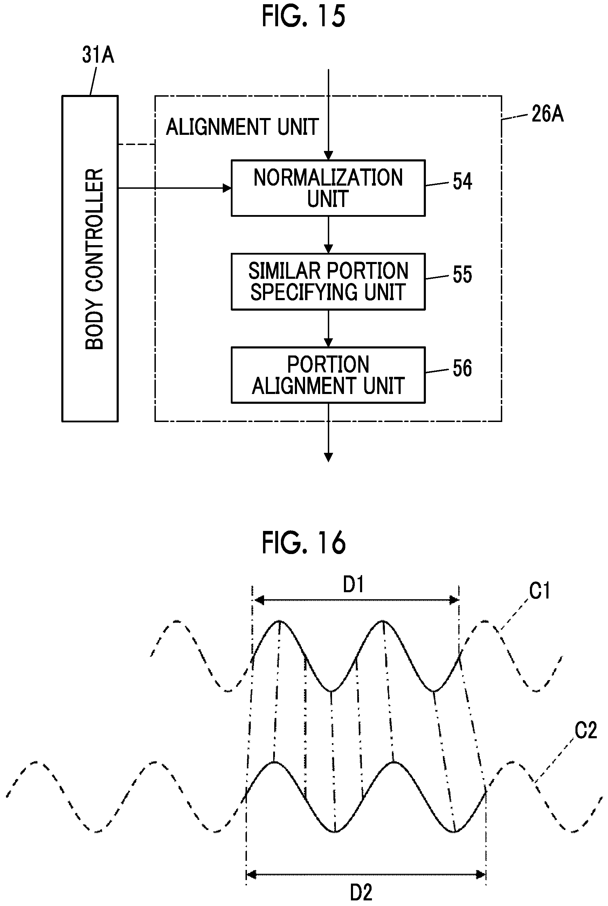

The alignment unit 26A aligns the first cross-sectional area change curve C1 and the second cross-sectional area change curve C2 with each other. As shown in Fig. 15, the alignment unit 26A has a configuration in which a normalization unit 54, a similar portion specifying unit 55, and a portion alignment unit 56 are connected in series.

The normalization unit 54 normalizes the entire first cross-sectional area change curve C1 and the entire second cross-sectional area change curve C2. The normalization unit 54 can normalize the first cross-sectional area change curve C1 and the second cross-sectional area change curve C2, for example, by dividing the first cross-sectional area change curve C1 and the second cross-sectional area change curve C2 using the maximum values thereof.

Further, the normalization unit 54 can normalize the first cross-sectional area change curve C1 and the second cross-sectional area change curve C2 by performing so-called Z-score normalization using the average value and the standard deviation of each of the first cross-sectional area change curve C1 and the second cross-sectional area change curve C2. According to the Z-score normalization, since the first cross-sectional area change curve C1 and the second cross-sectional area change curve C2 can be smoothed, even in a case in which there are many outliers in the first cross-sectional area change curve C1 and the second cross-sectional area change curve C2, that is, many frames in which the cavity of the heart H is erroneously extracted, the first cross-sectional area change curve C1 and the second cross-sectional area change curve C2 can be accurately aligned with each other.

As schematically shown in Fig. 16, the similar portion specifying unit 55 specifies the portions that are similar to each other in the normalized first cross-sectional area change curve C1 and the normalized second cross-sectional area change curve C2 as the first curved portion D1 and the second curved portion D2.

As a specific example, the similar portion specifying unit 55 divides a section from the start point in time to the end point in time of the first cross-sectional area change curve C1 into M parts at a regular time interval, and sets the first matrix X representing the normalized values in the first cross-sectional area change curve C1 corresponding to the M divided points in time as in Expression (1).

In addition, the similar portion specifying unit 55 divides a section from the start point in time to the end point in time of the second cross-sectional area change curve C2 into N parts at regular time intervals, and sets the second matrix Y representing the normalized values in the second cross-sectional area change curve C2 corresponding to the N divided points in time as in Expression (2).

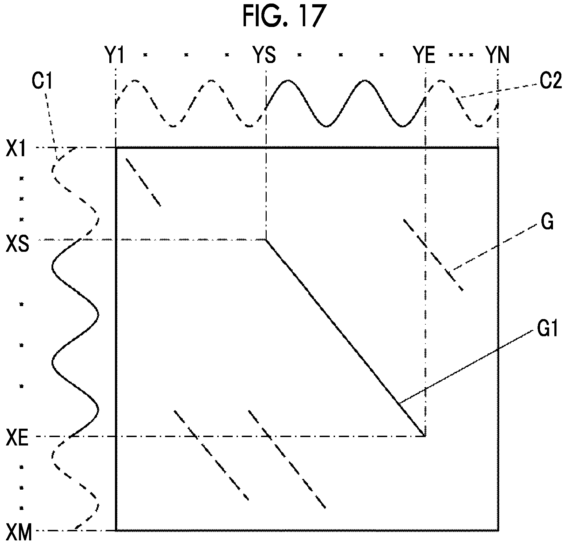

The similar portion specifying unit 55 creates, for example, the two-dimensional array as shown in Fig. 17 using each element of the first matrix X and each element of the second matrix Y. The similar portion specifying unit 55 considers that the element XL and the element YK are equal to each other in a case in which an absolute value |XL - YK| of a difference between the element XL of the first matrix X and the element YK of the second matrix Y is equal to or less than a predetermined threshold value, and calculates a plurality of routes G which proceeds in a direction from the element X1 to the element XM and in a direction from the element Y1 to the element YN in the two-dimensional array and in which the elements of the first matrix X and the elements of the second matrix Y are consecutively equal to each other, by using, for example, a dynamic programming method. Here, L is an integer of equal to or greater than 1 and equal to or less than M, and K is an integer of equal to or greater than 1 and equal to or less than N.

The similar portion specifying unit 55 specifies the longest route G1 among the plurality of calculated routes G, and specifies, as the first curved portion D1, a portion of the first cross-sectional area change curve C1 corresponding to a section from the element XS of the first matrix X at a starting point (XS, YS) of the route G1 to the element XE of the first matrix X at an end point (XE, YE) of the route G1. In addition, the similar portion specifying unit 55 specifies, as the second curved portion D2, a portion of the second cross-sectional area change curve C2 corresponding to a section from the element YS of the second matrix Y at the starting point (XS, YS) of the route G1 to the element YE of the second matrix Y at the end point (XE, YE) of the route G1. Here, S is an integer of equal to or greater than 1 and equal to or less than M, and E is an integer of equal to or greater than 1 and equal to or less than N.

In a case in which a plurality of longest routes G1 are calculated, the similar portion specifying unit 55 can specify, for example, the route G1 having a minimum sum of absolute values of differences between the elements of the first matrix X and the elements of the second matrix Y in the routes G1, that is, |XL - YK|, as a final route G1. The first curved portion D1 and the second curved portion D2 corresponding to the route G can be considered to be more similar to each other as the sum of the difference values |XL - YK| in the route G is smaller.

The portion alignment unit 56 aligns the first curved portion D1 and the second curved portion D2, which are specified by the similar portion specifying unit 55, with each other. The portion alignment unit 56 can align, for example, the point on the first curved portion D1 corresponding to (E-S+1)th divided point in time and the point on the second curved portion D2 corresponding to (E-S+1)th divided point in time as shown in Fig. 16 by associating the element XL and the element YK in the coordinates (XL, YK) of each point on the longest route G in the two-dimensional array created by the similar portion specifying unit 55 with each other.

In addition, the portion alignment unit 56 can also align the first curved portion D1 and the second curved portion D2 with each other by using an algorithm such as DTW, for example, as in the portion alignment unit 53 according to Embodiment 1.

As described above, with the alignment unit 26A according to Embodiment 2, the user does not need to perform the input operation to select the first curved portion D1 and the second curved portion D2, and thus the first curved portion D1 and the second curved portion D2 can be more easily aligned with each other.

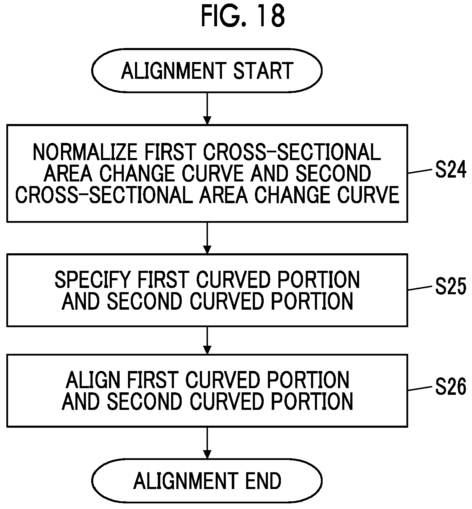

Next, processing of alignment performed by the alignment unit 26A will be described using a flowchart shown in Fig. 18.

First, in step S24, the normalization unit 54 of the alignment unit 26A normalizes the first cross-sectional area change curve C1 and the second cross-sectional area change curve C2 generated by the cross-sectional area change curve generation unit 25. In this case, the normalization unit 54 can normalize the first cross-sectional area change curve C1 and the second cross-sectional area change curve C2 by dividing the first cross-sectional area change curve C1 and the second cross-sectional area change curve C2 using the maximum values thereof. Further, the normalization unit 54 can normalize the first cross-sectional area change curve C1 and the second cross-sectional area change curve C2 by performing Z-score normalization on the first cross-sectional area change curve C1 and the second cross-sectional area change curve C2.

In step S25, the similar portion specifying unit 55 of the alignment unit 26A specifies the longest portions that are similar to each other in the first cross-sectional area change curve C1 and the second cross-sectional area change curve C2, which are normalized in step S24, as the first curved portion D1 and the second curved portion D2. The similar portion specifying unit 55 creates the two-dimensional array as shown in Fig. 17 using, for example, each element in a case in which the section of the first cross-sectional area change curve C1 is divided into M parts and each element in a case in which the section of the second cross-sectional area change curve C2 is divided into N parts. The similar portion specifying unit 55 specifies the longest route G1 in which the elements in the first cross-sectional area change curve C1 and the elements in the second cross-sectional area change curve C2 are consecutively equal to each other in the two-dimensional array. The similar portion specifying unit 55 can specify the portions corresponding to the specified route G in the first cross-sectional area change curve C1 and the second cross-sectional area change curve C2 as the first curved portion D1 and the second curved portion D2.

Finally, in step S26, the portion alignment unit 56 of the alignment unit 26A performs alignment between the first curved portion D1 and the second curved portion D2 specified in step S25. The portion alignment unit 56 can align, for example, the point on the first curved portion D1 corresponding to (E-S+1)th divided point in time and the point on the second curved portion D2 corresponding to (E-S+1)th divided point in time as shown in Fig. 16 by associating the element XL and the element YK in the coordinates (XL, YK) of each point on the longest route G in the two-dimensional array created by the similar portion specifying unit 55 with each other.

As described above, with the ultrasound diagnostic apparatus according to Embodiment 2, the alignment unit 26A automatically specifies the first curved portion D1 and the second curved portion D2 that are similar to each other in the first cross-sectional area change curve C1 and the second cross-sectional area change curve C2, so that the user does not need to perform the input operation to select the first curved portion D1 and the second curved portion D2, and thus the first curved portion D1 and the second curved portion D2 can be more easily aligned with each other.

Explanation of References

1: ultrasound probe

2, 2A: apparatus body
11: transducer array
12: transmission-and-reception circuit
21: image generation unit
22: display controller
23: monitor
24: cross-sectional area calculation unit
25: cross-sectional area change curve generation unit
26, 26A: alignment unit
27: memory
28: frame selection unit
29: frame update unit
30: measurement unit

31, 31A: body controller
32: input device
33: image acquisition unit
34, 34A: processor
41: pulser
42: amplifying unit
43: AD conversion unit
44: beam former
45: signal processing unit
46: DSC
47: image processing unit
51: curve selection unit
52, 54: normalization unit
53, 56: portion alignment unit
55: similar portion specifying unit
2C: apical two-chamber cross section
4C: apical four-chamber cross section
A1, A2: left ventricle
B, G, G1: route
B1: shortest route
C1: first cross-sectional area change curve
C2: second cross-sectional area change curve
D1: first curved portion
D2: second curved portion
H: heart
Q1, Q2: maximal value
R1, R2: minimal value
P1, P2: point
U1: first ultrasound image
U2: second ultrasound image
X1, XE, XM, XS, Y1, YE, YM, YS: element

**Claims**

1. An ultrasound diagnostic apparatus comprising:

   an image acquisition unit (33) that acquires first ultrasound images of a plurality of frames which are consecutive in time series and in which a first cross section of a heart of a subject is imaged, and second ultrasound images of a plurality of frames which are consecutive in time series and in which a second cross section of the heart different from the first cross section is imaged;
   a cross-sectional area calculation unit (24) that calculates a cross-sectional area of a cavity of the heart for each frame for the first ultrasound images of the plurality of frames and the second ultrasound images of the plurality of frames;
   a cross-sectional area change curve generation unit (25) that generates a first cross-sectional area change curve of the first cross section and a second cross-sectional area change curve of the second cross section, each of which represents a temporal change in the cross-sectional area, based on the cross-sectional area calculated by the cross-sectional area calculation unit;
   an alignment unit (26, 26A) that aligns the first cross-sectional area change curve and the second cross-sectional area change curve, which are generated by the cross-sectional area change curve generation unit (25), with each other;
   a monitor (23) that displays the first ultrasound image and the second ultrasound image at corresponding points on the first cross-sectional area change curve and the second cross-sectional area change curve; and
   a frame update unit (29) that, in a case in which one frame of the first ultrasound image or the second ultrasound image displayed on the monitor (23) is updated based on an instruction from a user, automatically updates the other frame of the first ultrasound image or the second ultrasound image so as to correspond with the one frame.

2. The ultrasound diagnostic apparatus according to claim 1,

wherein the alignment unit (26) includes

a curve selection unit (51) that selects a first curved portion and a second curved portion, which include the same number of change cycles, from the first cross-sectional area change curve and the second cross-sectional area change curve,
a normalization unit (52) that normalizes the first curved portion and the second curved portion, and
a portion alignment unit (53) that aligns the first curved portion and the second curved portion, which are normalized by the normalization unit (52), with each other.

3. The ultrasound diagnostic apparatus according to claim 2,
wherein the curve selection unit (51) selects the first curved portion and the second curved portion based on sections selected by the user in the first cross-sectional area change curve and the second cross-sectional area change curve.

4. The ultrasound diagnostic apparatus according to either claim 2 or claim 3,
wherein the curve selection unit (51)

selects the first curved portion or the second curved portion based on a section selected by the user in the first cross-sectional area change curve or the second cross-sectional area change curve,
calculates the number of change cycles included in one of the first curved portion or the second curved portion, and
selects the other of the first curved portion or the second curved portion based on the calculated number of change cycles.

5. The ultrasound diagnostic apparatus according to any one of claims 2 to 4,
wherein the curve selection unit (51) selects the first curved portion and the second curved portion which include the number of change cycles that is the same number as a designated number of cycles designated by the user.

6. The ultrasound diagnostic apparatus according to any one of claims 2 to 5,
wherein the normalization unit (52) normalizes the first curved portion and the second curved portion using respective maximum values thereof.

7. The ultrasound diagnostic apparatus according to any one of claims 1 to 6,
wherein the alignment unit (26A) includes

a normalization unit (54) that normalizes the first cross-sectional area change curve and the second cross-sectional area change curve,
a similar portion specifying unit (55) that specifies a first curved portion and a second curved portion which are portions most similar to each other in the first cross-sectional area change curve and the second cross-sectional area change curve which are normalized by the normalization unit (54), and
a portion alignment unit (56) that aligns the specified first curved portion and the specified second curved portion with each other.

8. The ultrasound diagnostic apparatus according to any one of claims 2 to 7,
wherein the normalization unit (54) normalizes the first cross-sectional area change curve and the second cross-sectional area change curve using respective maximum values thereof.

9. The ultrasound diagnostic apparatus according to any one of claims 2 to 7,
wherein the normalization unit (54) normalizes the first cross-sectional area change curve and the second cross-sectional area change curve using respective average values and standard deviations thereof.

10. The ultrasound diagnostic apparatus according to any one of claims 1 to 9, further comprising:
a frame selection unit (28) that selects, as a first measurement frame group, one set of the first ultrasound images corresponding to a maximal value and a minimal value in the first cross-sectional area change curve, and selects, as a second measurement frame group, one set of the second ultrasound images corresponding to a maximal value and a minimal value in the second cross-sectional area change curve aligned with the maximal value and the minimal value in the first cross-sectional area change curve by the alignment unit (26, 26A).

11. The ultrasound diagnostic apparatus according to claim 10,

wherein the monitor (23) displays the first ultrasound image of the first measurement frame group and the second ultrasound image of the second measurement frame group which are selected by the frame selection unit (28), and

in a case in which one frame of the first ultrasound image or the second ultrasound image displayed on the monitor is updated, the frame update unit (29) updates the other frame of the first ultrasound image or the second ultrasound image so as to correspond with the one frame.

12. A method of controlling an ultrasound diagnostic apparatus, the method comprising:

acquiring first ultrasound images of a plurality of frames which are consecutive in time series and in which a first cross section of a heart of a subject is imaged, and second ultrasound images of a plurality of frames which are consecutive in time series and in which a second cross section of the heart different from the first cross section is imaged;

calculating a cross-sectional area of a cavity of the heart for each frame for the first ultrasound images of the plurality of frames and the second ultrasound images of the plurality of frames;

generating a first cross-sectional area change curve of the first cross section and a second cross-sectional area change curve of the second cross section, each of which represents a temporal change in the cross-sectional area, based on the calculated cross-sectional area;

aligning the first cross-sectional area change curve and the second cross-sectional area change curve, which are generated, with each other;

displaying the first ultrasound image and the second ultrasound image at corresponding points on the first cross-sectional area change curve and the second cross-sectional area change curve; and

automatically updating, in a case in which one frame of the first ultrasound image or the second ultrasound image displayed on the monitor is updated based on an instruction from a user, the other frame of the first ultrasound image or the second ultrasound image so as to correspond with the one frame.

## FIG. 1

ULTRASOUND PROBE

TRANSDUCER ARRAY — 11

1

TRANSMISSION-AND-RECEPTION CIRCUIT — 12

2

APPARATUS BODY

IMAGE GENERATION UNIT — 33

21

31

34

CROSS-SECTIONAL AREA CALCULATION UNIT — 24

CROSS-SECTIONAL AREA CHANGE CURVE GENERATION UNIT — 25

ALIGNMENT UNIT — 26

BODY CONTROLLER

MEMORY — 27

28

FRAME SELECTION UNIT

29

FRAME UPDATE UNIT

MEASUREMENT UNIT — 30

DISPLAY CONTROLLER — 22

INPUT DEVICE — 32

MONITOR — 23

# FIG. 2

```
┌─────────────────────────────┐
│      TRANSDUCER ARRAY       │──── 11
└─────────────────────────────┘
```

┌─ TRANSMISSION ──────────────────────────────────┐ ── 12
│  -AND-RECEPTION
│  CIRCUIT
│
│   ┌──────────────┐      ┌──────────────────┐
│   │    PULSER    │      │  AMPLIFYING UNIT │── 42
│   └──────────────┘      └──────────────────┘
│          │                       │
│          41                      ▼
│                        ┌──────────────────┐
│                        │ AD CONVERSION UNIT│── 43
│                        └──────────────────┘
│                                  │
│                                  ▼
│                        ┌──────────────────┐
│                        │   BEAM FORMER    │── 44
│                        └──────────────────┘
└─────────────────────────────────┼──────────────┘
                                   ▼

# FIG. 3

┌─ IMAGE ────────────────────────────────────────┐ ── 21
│  GENERATION UNIT
│
│              ┌──────────────────────┐
│              │      SIGNAL          │── 45
│              │  PROCESSING UNIT     │
│              └──────────────────────┘
│                        │
│                        ▼
│              ┌──────────────────────┐
│              │        DSC           │── 46
│              └──────────────────────┘
│                        │
│                        ▼
│              ┌──────────────────────┐
│              │       IMAGE          │── 47
│              │  PROCESSING UNIT     │
│              └──────────────────────┘
└────────────────────────┼───────────────────────┘
                         ▼

## FIG. 4

H

A1

4C

U1

## FIG. 5

H

A2

2C

U2

## FIG. 6

D1

C1

CROSS-SECTIONAL AREA

TIME

# FIG. 7

# FIG. 8

## FIG. 9

## FIG. 10

## FIG. 11

# FIG. 12

```
            START

ACQUIRE FIRST ULTRASOUND IMAGE ─── S1

CALCULATE CROSS-SECTIONAL AREA
OF CAVITY OF HEART             ─── S2
IN FIRST ULTRASOUND IMAGE

GENERATE FIRST CROSS-SECTIONAL ─── S3
AREA CHANGE CURVE

        END OF ACQUISITION          S4
     N  OF FIRST ULTRASOUND
        IMAGE?
                                Y

ACQUIRE                        ─── S5
SECOND ULTRASOUND IMAGE

CALCULATE CROSS-SECTIONAL AREA
OF CAVITY OF HEART IN SECOND   ─── S6
ULTRASOUND IMAGE

GENERATE
SECOND CROSS-SECTIONAL AREA    ─── S7
CHANGE CURVE

        END OF ACQUISITION          S8
     N  OF SECOND ULTRASOUND
        IMAGE?
                                Y


ALIGN FIRST ULTRASOUND IMAGE   ─── S9
AND SECOND ULTRASOUND IMAGE

SELECT FIRST FRAME GROUP       ─── S10

SELECT SECOND FRAME GROUP      ─── S11

        FRAME UPDATE?                S12
                                N
                                Y

MANUALLY SELECT                ─── S13
FIRST ULTRASOUND IMAGE

AUTOMATICALLY SELECT           ─── S14
SECOND ULTRASOUND IMAGE

        END OF FRAME UPDATE?         S15
                                N
                                Y

EXECUTE MEASUREMENT            ─── S16

            END
```

25

## FIG. 13

ALIGNMENT START

SELECT FIRST CURVED PORTION
AND SECOND CURVED PORTION — S21

NORMALIZE FIRST CURVED PORTION
AND SECOND CURVED PORTION — S22

ALIGN FIRST CURVED PORTION
AND SECOND CURVED PORTION — S23

ALIGNMENT END

## FIG. 14

# FIG. 15

31A

ALIGNMENT UNIT                                    26A

BODY CONTROLLER

| NORMALIZATION UNIT | 54 |

| SIMILAR PORTION SPECIFYING UNIT | 55 |

| PORTION ALIGNMENT UNIT | 56 |

# FIG. 16

D1                                    C1

C2

D2

## FIG. 17

## FIG. 18

```
        ┌─────────────────────────┐
        │     ALIGNMENT START     │
        └─────────────────────────┘
                     │
                     ▼
   ┌──────────────────────────────────────┐
   │  NORMALIZE FIRST CROSS-SECTIONAL     │
   │  AREA CHANGE CURVE AND SECOND        │──── S24
   │  CROSS-SECTIONAL AREA CHANGE CURVE   │
   └──────────────────────────────────────┘
                     │
                     ▼
   ┌──────────────────────────────────────┐
   │   SPECIFY FIRST CURVED PORTION       │
   │   AND SECOND CURVED PORTION          │──── S25
   └──────────────────────────────────────┘
                     │
                     ▼
   ┌──────────────────────────────────────┐
   │    ALIGN FIRST CURVED PORTION        │
   │    AND SECOND CURVED PORTION         │──── S26
   └──────────────────────────────────────┘
                     │
                     ▼
        ┌─────────────────────────┐
        │      ALIGNMENT END      │
        └─────────────────────────┘
```

## EUROPEAN SEARCH REPORT

**Application Number**

EP 25 19 7266

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/044283 A1 (YONEYAMA NAOKI [JP]) 4 March 2004 (2004-03-04) | 1,12 | INV. A61B8/08 |
| Y | * paragraph [0053] - paragraph [0058] * * paragraph [0122] - paragraph [0124] * * paragraph [0127] - paragraph [0155] * * figures 2,10-15 * | 2-11 | A61B8/00 G06T7/00 G16H50/30 |
| Y | US 2002/123688 A1 (YAMAUCHI MASAKI [JP]) 5 September 2002 (2002-09-05) * paragraph [0108] - paragraph [0121] * * paragraph [0132] - paragraph [0135] * * figures 8-10 * | 2-11 | |

----- 

-----

**TECHNICAL FIELDS SEARCHED (IPC)**

A61B
G06T
G16H

The present search report has been drawn up for all claims

| Place of search | Date of completion of the search | Examiner |
|---|---|---|
| Munich | 21 January 2026 | De la Hera, Germán |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 19 7266

This annex lists the patent family members relating to the patent documents cited in the above-mentioned European search report.
The members are as contained in the European Patent Office EDP file on
The European Patent Office is in no way liable for these particulars which are merely given for the purpose of information.

21-01-2026

| Patent document cited in search report | Publication date | Patent family member(s) | Publication date |
|---|---|---|---|
| US 2004044283 A1 | 04-03-2004 | CN 1486675 A | 07-04-2004 |
| | | US 2004044283 A1 | 04-03-2004 |
| US 2002123688 A1 | 05-09-2002 | US 2002123688 A1 | 05-09-2002 |
| | | US 2004176689 A1 | 09-09-2004 |
| | | US 2004215078 A1 | 28-10-2004 |

EPO FORM P0459

For more details about this annex : see Official Journal of the European Patent Office, No. 12/82

# EP 4 706 547 A1

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004073850 A